Europäisches Patentamt

European Patent Office

Office européen des brevets

(11) Veröffentlichungsnummer : **0 128 233**
**B1**

(12) **EUROPÄISCHE PATENTSCHRIFT**

(45) Veröffentlichungstag der Patentschrift :
27.05.87

(51) Int. Cl.⁴ : **A 23 L   2/00**, A 61 K 35/08,
**C 02 F   1/44**, B 01 D 13/00

(21) Anmeldenummer : 83105780.7

(22) Anmeldetag : 13.06.83

(54) Verfahren zur Herstellung von Mineral- und Heilwasserprodukten gezielter Zusammensetzung.

(43) Veröffentlichungstag der Anmeldung :
19.12.84 Patentblatt 84/51

(45) Bekanntmachung des Hinweises auf die Patenterteilung : 27.05.87 Patentblatt 87/22

(84) Benannte Vertragsstaaten :
AT DE IT

(56) Entgegenhaltungen :
DE-A- 2 032 863
DE-A- 2 615 222

(73) Patentinhaber : **KÖZPONTI ELELMISZERIPARI**
**KUTATO INTEZET**
**Herman Otto ut 15**
**H-1022 Budapest (HU)**

(72) Erfinder : **Khell, Adámné**
**Fiáth János u. 16**
**H-1015 Budapest (HU)**
Erfinder : **Demeczky, Mihály, Dr.**
**Hegyalja ut 70**
**H-1112 Budapest (HU)**
Erfinder : **Godek, Ferencné**
**Czetz J. u. 19**
**H-1039 Budapest (HU)**
Erfinder : **Kustár, Lásio**
**Társasház u. 5**
**H-1225 Budapest (HU)**

(74) Vertreter : **Hansen, Bernd, Dr.rer.nat. et al**
**Hoffmann, Eitle & Partner Patentanwälte Arabella-**
**strasse 4**
**D-8000 München 81 (DE)**

**Beschreibung**

Die Erfindung betrifft ein Verfahren zur Herstellung von Mineral- und Heilwasserprodukten gezielter Zusammensetzung.

Eine in gewünschten Grenzen zur Standardisierung der Zusammensetzung von Mineral- und Heilwasserprodukten geeignete Methodik ist bekannt und wird auch heute in der Praxis angewandt. Sie beruht auf der Ausnutzung der Tatsache, dass sich die einzelnen Wasserschichten entsprechend der jeweiligen Konzentration aufgrund des spezifischen Gewichtes trennen. Eine andere bekannte Methodik zur Erreichung des gewünschten Konzentrationsbereiches stellt die Verdampfung dar.

Die Ausnutzung der jeweiligen spezifischen Gewichte sichert die « originelle », natürliche und den Wassertyp charakterisierende Ionenzusammensetzung. Für deren Anwendung gibt es aber immer weniger Möglichkeiten, da wegen der Erschöpfung der Brunnen (die Verminderung der gelösten Materialien bzw. die der Konzentration) die austreibbare, der vorgeschriebenen Zusammensetzung entsprechende Wassermenge immer mehr abnimmt. Als Wirkung des regenreichen Wetters vermindert sich auch die Salzkonzentration der Wasserschichten. So kann in einigen Fällen, wie z. B. Bitterwasser Hunyadi János, Igmándi, Apenta, Mira usw., statistisch nur 1/5 der gesamten Vollwassermenge des Brunnens in Flaschen abgezogen werden, während 4/5 abfliessen, was auch zu Umweltschutzproblemen führen kann. Die erhebliche Kosten mit sich bringenden neuen Brunnensiedlungen führen nur zur früheren Erschöpfung der das Mineral- und Heilwasser liefernden Bodenschichten.

Die Einstellung der gewünschten Konzentration der im Mineral- und Heilwasser gelösten Materialien durch Verdampfung stellt eine sehr energieintensive Methodik dar une bringt in den meisten Fällen die Abänderung der originellen natürlichen Konzentration mit sich. So zerfallen z. B. die Hydrokarbonate, entweichen gegebenenfalls gewisse Komponenten (Halogene, Emanationen) und es kommt zur Ausscheidung einiger Salzkomponenten. Darüber hinaus führt der Hitzeeffekt zu einem unangenehmen Trinkaroma (« gekochtes Aroma »).

Zur Beseitigung der vorher geschilderten Nachteile wird gemäss der Erfindung gegenüber den auch heutzutage üblichen, die Konzentrationsgrenzen einstellenden Verfahren ein physikalisches Verfahren angewandt, in dem die volle Anwendung der Vollwassermenge des Brunnens ohne Hitzeeffekt ermöglicht wird. Dabei ist nur ungefähr 1/5 des Energieaufwandes im Verhältnis zu dem Verdampfungsverfahren bei gleichzeitiger Sicherung der das Mineral- und Heilwasser charakterisierenden Ionenzusammensetzung erforderlich. Zum ersten Mal wurde aufgrund der vorliegenden Erfindung die Membranseparationstechnik zur Konzentrierung von Mineral- und Heilwasser verschiedener Type bzw. zur Einstellung der gewünschten Konzentrationsgrenzen angewendet wie auch zur Herstellung von Heilwässern aus den nur Mineralwasser liefernden Brunnen, ohne Störung und Zerstörung des natürlichen Ionenmilieus.

Die auf Membranseparation beruhenden Konzentrationsverfahren basieren im Wesen auf dem Prinzip der Osmose bei Lösungen. Wenn ein reines Lösungsmittel und eine Lösung, oder zwei Lösungen verschiedener Konzentration durch eine semipermeable Membran voneinander getrennt sind, welche nur für die Moleküle des Lösungsmittels durchlässig ist, dann strömen die Lösungsmittelmoleküle solange durch die Membran in die Lösung grösserer Konzentration, bis der Gleichgewichtszustand eingestellt ist. Eine gegenseitige Lösungsmittelströmung erfolgt, wenn ein den Osmosendruck der konzentrierteren Lösung übersteigender Druck auf dieselbe eine Wirkung ausübt. Diese letztere Möglichkeit wird bei den Membranseparationsverfahren (Hyperfiltration, Ultrafiltration, usw.), zur teilweisen Entfernung des überflüssigen Lösungsmittels (meistens Wasser) bzw. zur Konzentrierung von Lösungen angewandt.

Die Membranseparationsvorrichtungen stellen Systeme mit grossem Druck dar. Die am häufigsten angewandten Membranen sind aus Zelluloseacetat hergestellt. Darüber hinaus können aber auch andere Polymere zum Zweck der Membranseparationen angewendet werden.

Das Wesen des erfindungsgemässen Verfahrens ist, dass das Mineral- oder Heilwasser, welches zur Abziehung in Flaschen nicht ausreichend konzentriert ist, unter einem Druck von 1 bis 12, vorzugsweise 4 bis 10 MPa bis zu der vorgeschriebenen Ionenkonzentration einer Hyperfiltration unterworfen und dann, gegebenenfalls mit der üblichen Technologie, in Flaschen abgezogen wird.

Die Einstellung der zur Abziehung in Flaschen notwendigen Ionenkonzentration kann gemäss dem Verfahren der Erfindung auch so geschehen, dass durch Hyperfiltration ein Konzentrat hergestellt wird. Mit demselben wird das Mineral- oder Heilwasser — welches wegen seiner geringen Konzentration nicht in Flaschen abgezogen werden kann — im entsprechenden Verhältnis gemischt, wobei die zur Abziehung in Flaschen notwendige Ionenkonzentration auf diesem Wege eingestellt wird.

Zweckmässig kann das Verfahren zur Erhöhung der gelösten Materialien einzelner Wassertypen angewendet werden, die wegen ihrer geringen Wirkstoffkonzentration als Mineralwasser bezeichnet sind. Dadurch können diese Wässer als Heilwasser qualifiziert werden.

Das erfindungsgemässe Verfahren ist zur Sicherung eines solchen Konzentrates geeignet, welches ohne irgendeine Konservierung gelagert bzw. in kleinem Rauminhalt geliefert werden kann und es ermöglicht durch seine im entsprechenden Verhältnis durchgeführte Mischung die Standardisierung der durch die Wasserergiebigkeit und andere Faktoren qualitativ und quantitativ beeinflussten Zusammensetzung. Diese können auch alternativ als Ausgangsstoffe für die Verdünnung zur Bildung von Mineral- oder Heilwasser gebraucht und vertrieben werden.

Die Erfindung ermöglicht eine bedeutende Energieeinsparung gegenüber der Herstellung von Heilsalzen durch ein Verdampfungsverfahren.

Das erfindungsgemässe Verfahren bietet eine Möglichkeit zur Einstellung des Verhältnisses der ein- bzw. mehrwertigen Ionen, entsprechend den Heilansprüchen bzw. Heilzwecken.

Zur Verwirklichung des Verfahrens kann jeder in sich selbst bekannte Hyperfiltrationsvorrichtungstyp angewendet werden. Es ist besonders zweckmässig, im Laufe der Konzentrierung periodisch oder fortlaufend eine Ultraschallbehandlung anzuwenden, welche die Ausbildung der Ansätze verhindert und zur Aufrechterhaltung der Durchlässigkeit der Membranen auf einem gewissen Niveau bzw. zur Erhöhung desselben beiträgt.

Das erfindungsgemässe Verfahren wird anhand der folgenden Beispiele illustriert :

Beispiel 1

Aus taubem Wasser mit einem gelösten Materialgehalt von 10-20.000 mg $dm^{-3}$, das aber die Ionen in dem der originalen Heilwirkung entsprechenden Verhältnis enthält, wird ein Heilwasser mit einer Konzentration von 20-25.000 mg $dm^{-3}$ in der folgenden Weise hergestellt :

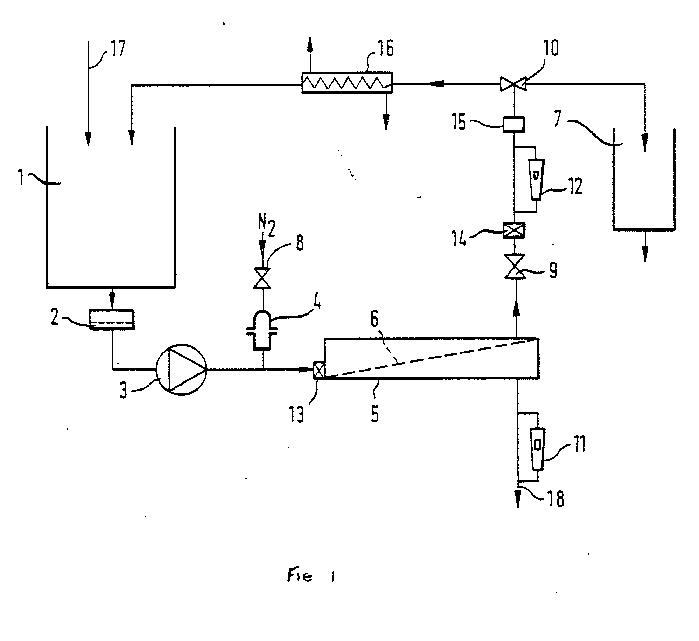

Es wird ein Brunnen herangezogen, welcher täglich 1 $m^3$ zur Abziehung in Flaschen geeignetes und ungefähr 4 $m^3$ durchschnittlich mit einer Konzentration von 10-12.000 mg $dm^{-3}$ taubes Wasser ergibt. Es soll nun die Menge an taubem Wasser von 4 $m^3$ auf 2 $m^3$ konzentriert werden. Die zur Konzentrierung geeignete Vorrichtung (Fig. 1) ist mit einer über eine Leistungsfähigkeit von 5 $m^2$ 400 $dm^3$ $m^{-2}$ verfügende Hyperfiltermembrane, die zweckmässig aus Zelluloseacetat gebildet ist, versehen. Die 4 $m^3$ taubes Wasser werden in den Zuführungsbehälter 1 geleitet. Sodann werden die mechanischen Verunreinigungen mit einem Vorfilter 2 entfernt und das Wasser wird durch eine Hochdruckpumpe 3 mit der Dazwischenschaltung eines Druckausgleichers 4 in die die Membranen beinhaltende Einheit 5 geführt. Aus dem auf die aktiven Oberflächen der Zelluloseacetat-Membranen 6 gepressten tauben Wasser wird ein Teil des Wassers durch die Membranen entfernt, der praktisch als salzfreies Filtrat unter atmosphärischem Druck abfliessen kann (18). Die die gelösten Ionen enthaltende konzentrierte Fraktion (Konzentrat) kann, durch das Reduktionsventil 9 unter Einschaltung einer das Leitfähigkeitsmessgerät 15 und den Verteilungshahn 10 regulierenden Einheit 15 entweder durch den Kühler 16 für die Rezirkulation in den Zuführungsbehälter 1 oder in den zur Sammlung des Konzentrates dienenden Behälter 7 geführt werden.

Einen Teil der Vorrichtung bilden weiter das Hochdruckventil 8, die Rotameter 11 und 12, der hydrodynamische Ultraschallstrahler 13 und der elektrodynamische Ultraschallstrahler 14. Der Pfeil 17 bedeutet die Zufuhr des Speisewassers. Mit der Inbetriebnahme einer über durchschnittliche Durchlässigkeit verfügende Membran 30 $m^2$ 400 $dm^3$ $m^{-2}$ (z. B. KEKI Nr. 73/28) und mit einem Druck von 4,0 MPa, wird ein Konzentrat von 2 $m^3$ (eine Konzentration von 20-25.000 mg $dm^{-3}$) gewonnen, welches nach üblicher Abziehung in Flaschen vertrieben werden kann.

Beispiel 2

Das für eine Abziehung in Flaschen ungeeignete, über zu geringe Konzentration verfügende Bitterwasser « Apenta » beinhaltet 3 232 mg $dm^{-3}$ $Na^+$ + $K^+$, 484 mg $dm^{-3}$ $Ca^{2+}$, 2 351 mg $dm^{-3}$ $Mg^{2+}$, 610 mg $dm^{-3}$ $Cl^-$, 15 684 mg $dm^{-3}$ $SO_4^{2-}$ und 872 mg $dm^{-3}$ $HCO_3^-$ vor der Konzentrierung, d. h. 6 067 mg $dm^{-3}$ Kationen und 17 166 mg $dm^{-3}$ Anionen. Der gesamte gelöste Materialinhalt desselben war 23 233 mg $dm^{-3}$. Nach einer durch Hyperfiltration bei Raumtemperatur durchgeführten 4,54-maligen Konzentrierung beträgt der $Na^+$ + $K^+$-Gehalt des Wasser 12 120, der $Ca^{2+}$-Gehalt 660, der $Mg^{2+}$-Gehalt 9 651, der $Cl^-$-Gehalt 2 092, der $SO_4^{2-}$-Gehalt 59 903 und der $HCO_3^-$-Gehalt 2 904 mg $dm^{-3}$. Die Konzentration der Kationen beträgt 22 431 mg $dm^{-3}$, während die der Anionen auf 64 898 mg $dm^{-3}$ geändert wurde. Die Konzentration der geprüften gesamten gelösten Materialien betrug 87 329 mg $dm^{-3}$. Das im Laufe der Konzentrierung entfernte Wasser (Filtrat) beinhaltet durchschnittlich insgesamt 1 454 mg gelöstes Ion pro $dm^3$. Infolge der Zusammensetzung und Konzentration des Filtrates war dasselbe für die Einstellung des zur Abziehung in Flaschen notwendigen Niveaus der gelösten Materialien bei den Heilwassertypen « Apenta » geringer Konzentration geeignet.

Unter gleichen Versuchsverhältnissen wurde der Inhalt der gesamten gelösten Materialien des Heilwassers « Hunyadi János » von 22 939 mg $dm^{-3}$ auf 110 264, des Heilwassers « Igmándi » von 24 125 mg $dm^{-3}$ auf 179 251 und des Heilwassers « Mira » von 16 841 auf 59 540 erhöht. Alle Konzentrate sind für die Einstellung der zur Abziehung in Flaschen notwendigen Konzentration geeignet.

Beispiel 3

Die in Beispiel 2 beschriebenen Konzentrate und die auf einem Druck von 8,0 bis 10,0 MPa mit einem grösseren Konzentrationsfaktor hergestellten Heilwasserkonzentrate sind nach Filtration und aseptischer Abziehung für die raumsparende Lagerung und für eine betriebsmaterialsparende Lieferung geeignet. Die gelagerten Konzentrate können als Reserve zur Standardisierung der vom

Wetter abhängigen Ionenkonzentration angewendet werden. Aus denselben kann man mit Wasser entsprechender Qualität verwässert ein Heilwasser mit einer mit dem Typ des natürlichen Ausgangswassers ähnlichen Konzentration herstellen. Im Einzelhandel sind diese auch für den unmittelbaren Verbrauch — gemäss dem gegebenen Rezept — nach Zurückverdünnung geeignet.

**Patentansprüche**

1. Verfahren zur Herstellung von für die Abfüllung in Flaschen bestimmten Mineral- bzw. Heilwasser gezielter Zusammensetzung, dadurch gekennzeichnet, daß das Mineral- bzw. Heilwasser bei einem Druck von 1 bis 12 MPa einer Hyperfiltration unterworfen wird, wobei dann das entstandene Produkt erhöhter Ionenkonzentration gegebenenfalls entsprechend verdünnt wird.

2. Verfahren nach Anspruch 1, dadurch gekennzeichnet, dass die Hyperfiltration bei einem Druck von 4 bis 10 MPa durchgeführt wird.

3. Verfahren nach Anspruch 1 oder 2, dadurch gekennzeichnet, dass die Verdünnung mit einer weiteren Menge des ursprünglich zur Konzentrierung angewendeten Mineral- oder Heilwasser durchgeführt wird.

**Claims**

1. Process for the production of mineral or medicinal water having a specific composition for bottling, characterized in that the mineral or medicinal water is subjected to hyperfiltration at a pressure of 1 to 12 MPa and in that the product having increased ion concentration obtained is then correspondingly diluted if necessary.

2. Process according to claim 1 characterized in that the hyperfiltration is carried out at a pressure of 4 to 10 MPa.

3. Process according to claim 1 or 2 characterized in that the dilution is carried out with a further quantity of the mineral or medicinal water originally used for concentration.

**Revendications**

1. Procédé de préparation d'eaux minérales ou médicinales, destinées à la mise en bouteilles et ayant une composition désirée, caractérisé en ce que l'on soumet l'eau minérale ou médicinale à une hyperfiltration sous une pression de 1 à 12 MPa, le produit résultant, de concentration ionique augmentée, étant ensuite éventuellement dilué de façon appropriée.

2. Procédé conforme à la revendication 1, caractérisé en ce que l'on effectue l'hyperfiltration sous une pression de 4 à 10 MPa.

3. Procédé conforme à la revendication 1 ou 2, caractérisé en ce que l'on effectue la dilution avec une autre quantité de l'eau minérale ou médicinale utilisée initialement pour la concentration.

Fie 1